(19) Europäisches Patentamt
European Patent Office
Office européen des brevets

(11) Veröffentlichungsnummer: **0 057 664**
**A2**

# (12) EUROPÄISCHE PATENTANMELDUNG

(21) Anmeldenummer: **82810028.9**

(22) Anmeldetag: **25.01.82**

(51) Int. Cl.³: **C 07 D 498/04**, C 12 P 17/18, C 12 N 1/20, A 61 K 31/42 // (C12N1/20, C12R1/48),(C07D498/04, 263/00, 205/00)

(30) Priorität: **30.01.81 CH 616/81**

(43) Veröffentlichungstag der Anmeldung: **11.08.82 Patentblatt 82/32**

(84) Benannte Vertragsstaaten: **AT BE CH DE FR GB IT LI NL SE**

(71) Anmelder: **CIBA-GEIGY AG, Patentabteilung Postfach, CH-4002 Basel (CH)**

(72) Erfinder: **Zähner, Hans, Prof.Dr., Im Hopfengarten 13, D-7400 Tübingen (DE)**
Erfinder: **Wanning, Martin, Dr., In der Hauptstrasse 42, D-6530 Bingen/Sponsheim (DE)**
Erfinder: **Krone, Bernd, Dr., Goetheallee 10, D-3400 Göttingen (DE)**
Erfinder: **Zeeck, Axel, Prof.Dr., Schlözerweg 7, D-3400 Göttingen (DE)**
Erfinder: **Peter, Heinrich, Dr., Bündtenweg 69, CH-4102 Binningen (CH)**

## (54) Clavam-Derivate.

(57) Die Erfindung betrifft das neue durch Fermentation eines neuen Mikroorganismus gebildete Antibiotikum «Tü 1718» und seine Komponenten und Derivate, insbesondere seine Hauptkomponente, eine Clavamverbindung der folgenden Formel

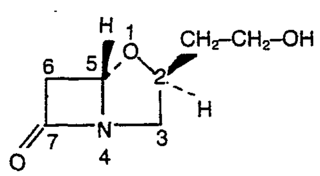

(I)

und ihre Derivate, wie Ester, Aether, Thioester und Thioäther. Beim neuen Mikroorganismus handelt es sich um den Stamm Tü 1718 der Art Streptomyces antibioticus, subsp. antibioticus, welcher aus einer Bodenprobe aus Nordthailand isoliert wurde.

Das neue Antibiotikum und seine Komponenten und Derivate besitzen antibakterielle, antifungische und tumorhemmende Wirkung.

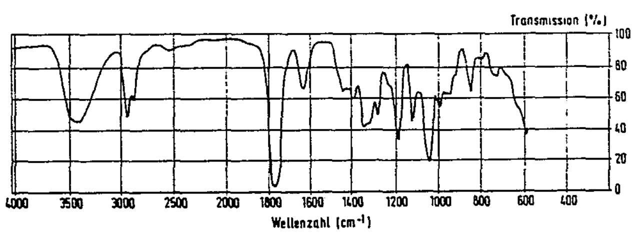


EP 0 057 664 A2

CIBA-GEIGY AG
Basel (Schweiz)

4-13270 /+

## Clavam-Derivate

Die Erfindung betrifft ein neues durch Fermentation gebildetes Antibiotikum und seine Komponenten und Derivate, insbesondere seine Hauptkomponente, ein neues Clavamderivat, sowie Verfahren zur Herstellung dieser Stoffe, Derivate der Hauptkomponente und ihre Herstellungsverfahren, pharmazeutische Präparate, die solche Verbindungen enthalten und die Verwendung dieser Verbindungen als Antibiotika oder als Zwischenprodukte.

Das neue durch Fermentation erhältliche Antibiotikum wird hier durch die Bezeichnung "Antibiotikum Tü 1718" charakterisiert. Seine Hauptkomponente $A_1$ stellt das Clavamderivat der Formel

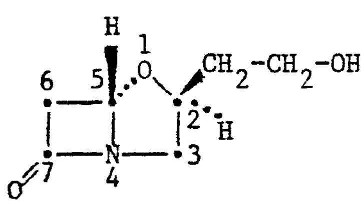

(I)

dar, also ein Clavam mit spezifischer Konfiguration an den zwei vorhandenen Asymmetriezentren, das unter Zugrundelegung der für das Grundgerüst "Clavam" vorgesehenen Numerierung als (2S,5S)-2-(2-Hydroxyäthyl)-clavam bezeichnet wird. Wie ersichtlich ist das H-Atom am 5 C-Atom in Bezug auf die Ringebene des Viererrings in β-Stellung und dasjenige am 2 C-Atom in α-Stellung, was den absoluten Konfigurationen S an beiden C-Atomen entspricht.

Die Zuordnung von Formel (I) zur Hauptkomponente erfolgte aufgrund von Massen -, $^{13}$C-NMR -, $^{1}$H-NMR- und Hochfrequenz- Protonen - Resonanz-Spektrum, sowie Zirkular Dichroismus und anderen physikalischen und chemischen Untersuchungen.

Das obige Clavam-Gerüst kann unter Zugrundelegung der IUPAC-Nomenklatur als 4-Oxa-1-azabicyclo[3,2,0]heptan-7-on bezeichnet werden, wobei dann die Numerierung der Ringatome folgendermassen ausfällt:

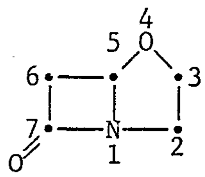

(II)

Die obigen Verbindungen der Formel (I) sind somit nach diesem Benennungssystem und unter Berücksichtigung der Konfiguration an den beiden asymmetrischen C-Atomen als (3S,5S)-3-(2-Hydroxyäthyl)-4-oxa-1-azabicyclo[3,2,0]heptan-7-on und seine Derivate mit funktionell abgewandelter Hydroxygruppe anzusprechen.

Im folgenden wird der ersten oben angegebenen Nomenklatur, die sich vom Clavamgrundgerüst

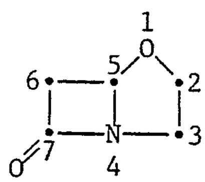

(III)

ableitet, der Vorzug gegeben. Die durch funktionelle Abwandlung der Hydroxygruppe im Clavamderivat der obigen Formel auf chemischem Wege herstellbaren funktionellen Derivate besitzen ebenfalls antibiotische Aktivität und gehören ebenfalls zum Gegenstand der vorliegenden Erfindung.

Die vorliegende Erfindung betrifft daher insbesondere die Clavamderivate der Formel

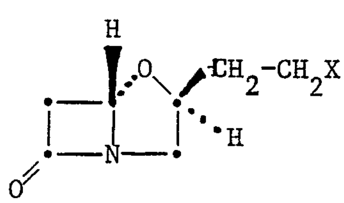

(IV)

in denen X eine freie oder funktionell abgewandelte Hydroxygruppe bedeutet, und gegebenenfalls Salze dieser Verbindungen, Verfahren zu ihrer Herstellung, sowie pharmazeutische Präparate mit einem Gehalt an solchen Verbindungen, und die Verwendung dieser Verbindungen oder pharmazeutischen Präparate als antifungische-, antibakterielle- und tumorhemmende Mittel.

Unter den Verfahren zur Herstellung der Verbindungen der Formel (IV) ist insbesondere das mikrobiologische, fermentative Verfahren zur Herstellung des neuen Antibiotikums Tü 1718 hervorzuheben, aus dem die Hauptkomponente (2S,5S)-2-(2-Hydroxyäthyl)-clavam isoliert werden kann. Diese Verbindung ist sowohl im Hinblick auf ihre antibiotische Wirkung wie auf ihre Verwendungsmöglichkeit zur Herstellung der oben genannten anderen antibiotisch wirksamen funktionellen Derivate von Bedeutung.

In der deutschen Offenlegungsschrift 2 702 091 sind Clavamderivate der Formel

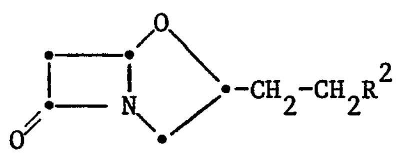

offenbart, worin $R^2$ unter anderem auch eine gegebenenfalls veresterte oder verätherte Hydroxygruppe sein kann. Auf Seite 6 jenes Patentes wird zwar behauptet, dass die Verbindungen mit der Konfiguration gemäss der dort wiedergegebenen Formel (IV) bevorzugt sind, die somit optische Antipoden zum Typ der Verbindungen der vorliegenden Anmeldung darstellen, jedoch geht aus der nachfolgenden Beschreibung, insbesondere der Herstellungsmethoden und der Kennzeichnung der Verfahrensprodukte hervor, dass nur Diastereomerengemische und Racemate erhalten wurden. Optisch aktive Verbindungen werden nirgends offenbart und mit der obengenannten Formel (IV) wurde offenbar lediglich die Racemat-Form des einen Diastereomeren-Typs gemeint, wie dem Text zu entnehmen ist.

Demgegenüber betrifft die vorliegende Erfindung die fermentative Darstellung reiner, an beiden Chiralitätszentren konfigurativ wohl definierter, Stereoisomeren (mit der der Formel IV entgegengesetzten Konfiguration). Die Verbindungen der obigen Formel (IV) der vorliegenden Erfindung zeichnen sich gegenüber den genannten bekannten Gemischen von Diastereomeren und Racematen durch eine erhöhte antibiotische und antifungische Wirksamkeit und bessere Verträglichkeit aus.

Ein weiterer Gegenstand der Erfindung ist der im folgenden noch näher beschriebene Mikroorganismen-Stamm, mit welchem die fermentative Herstellung der genannten neuen Verbindungen möglich gewesen ist und Mutanten desselben, die z.B. durch bekannte chemische oder physikalische Methoden herstellbar sind, und die ebenfalls das neue Antibiotikum Tü 1718, gegebenenfalls mit erhöhter Ausbeute, zu produzieren vermögen.

Schliesslich gehören zum Gegenstand der Erfindung auch die das neue Antibiotikum Tü 1718 enthaltenden Züchtungen oder Fermentationsbrühen, und die daraus, wie im folgenden beschrieben, erhältlichen Extrakte.

In Verbindungen der Formel (IV) kann X eine funktionell abgewandelte Hydroxygruppe sein. Als solche sind insbesondere verätherte und veresterte Hydroxygruppen zu verstehen.

Eine verätherte Hydroxygruppe ist z.B. eine gegebenenfalls substituierte aliphatische, carbocyclische oder carbocyclisch-aliphatische Hydrocarbyloxy- oder Heterocyclyloxy-Gruppe mit 1-12 C-Atomen, insbesondere eine Alkoxygruppe mit 1-7 C-Atomen, vor allem eine Niederalkoxy, wie eine Aethoxy- oder Methoxygruppe.

Eine veresterte Hydroxygruppe X kann sich von einer organischen oder anorganischen Säure ableiten. Von den anorganischen Säuren sind z.B. Schwefel- und Phosphorsäuren zu nennen. Sodann sind unter den anorganischen Estergruppen auch die Ester mit den Halogenwasserstoffsäuren zu zählen, d.h. die (2S,5S)-2-(2-Halogenäthyl)-clavame, wie insbesondere das (2S,5S)-2-(2-Chloräthyl- und 2-Bromäthyl)-clavam.

In Estergruppen, die sich von organischen Säuren ableiten, können die Kohlenwasserstoffreste unsubstituiert oder durch Halogene, z.B. Chlor oder Brom, veresterte oder verätherte Hydroxygruppen, Amino- oder Carboxylgruppen substituiert sein. Verestertes Hydroxy, das sich von einer organischen Carbonsäure oder Dicarbonsäure ableitet, ist vor allem aliphatisches Acyloxy mit 1-12 C-Atomen, insbesondere Alkanoyloxy, vor allem gegebenenfalls durch vorzugsweise 1-2 Halogenatome, insbesondere Chlor oder Brom, Hydroxy-, Alkoxy- oder Aryloxygruppen, wie Phenoxygruppen, substituiertes Niederalkanoyloxy, wie z.B. Formyloxy, Acetoxy, Propionoxy, Butyryloxy, 2-Chloracetoxy, 2,2-Dichloracetoxy, 3,3-Dichlorpropionyloxy, 2-Hydroxyacetoxy, Hydroxycarbonyloxy oder eine Niederalkoxycarbonyloxy-, oder eine unsubstituierte oder im aromatischen Teil durch 1-2 Chloratome, insbesondere Chlor oder Brom, Methylgruppen, Aminogruppen oder Nitrogruppen substituierte Phenoxycarbonyloxy-Gruppe, wie 2-Methoxyacetoxy oder 2-Phenoxyacetoxy. Die Acyloxygruppe kann sich aber auch von einer carbocyclischen, z.B. einer aromatischen, aromatisch-aliphatischen oder heterocyclischen organischen Säure, ableiten und kann somit Cycloalkanoyloxy, Phenylcarbonyloxy oder Phenyl-niederalkanoyloxy sein. Besonders hervorzuheben sind auch niederaliphatische Urethane mit 1-7 C-Atomen, wie z.B. das Methyl-, Aethyl- oder n-Propylurethan oder unsubstituiertes oder z.B. durch 1-2 Methylgruppen oder 1-2 Chlor- oder Bromatome substituiertes Phenylurethan.

Ester von organischen Säuren können sich aber auch von Sulfonsäuren ableiten, z.B. monocyclischen aromatischen oder niederaliphatischen Sulfonsäuren.

Der Heterocyclyl-Rest einer sich von einer heterocyclischen Carbonsäure ableitenden Estergruppe oder einer Aethergruppe ist vorzugsweise ein gegebenenfalls substituiertes, gesättigtes oder ungesättigtes monocyclisches, fünf- oder sechsgliedriges Heterocyclyl mit einem Stickstoff-, Sauerstoff- oder Schwefelatom als Ringglied.

Unter verätherten und veresterten Hydroxygruppen X können aber auch die sich von Thiolen bzw. Thiosäuren abgeleiteten verstanden werden, wobei vorzugsweise jene Thiole bzw. Thiosäuren in Betracht kommen, die den oben besonders hervorgehobenen Alkoholen und Säuren als Aether- bzw. Esterkomponenten entsprechen, wie z.B. eine Niederalkylthio-, z.B. Methyl- oder Aethylthiogruppe, bzw. eine Acylthio-, z.B. eine Niederalkylcarbonylthio-, in erster Linie die Formylthio- und Aethylthiogruppe.

Die vor- und nachstehend verwendeten Allgemeindefinitionen haben im Rahmen der vorliegenden Beschreibung vorzugsweise die folgenden Bedeutungen:

"Nieder-" umschreibt im Zusammenhang mit obigen aliphatischen Hydrocarbyl und insbesondere Alkoxy- und Alkanoyloxygruppen einen Rest mit 1-7 C-Atomen. Der Niederalkylrest ist in solchen Gruppen z.B. Methyl, Aethyl, n-Propyl, Isopropyl, n-Butyl, Isobutyl, sek.-Butyl oder tert.-Butyl, ferner n-Pentyl, Neopentyl, n-Hexyl oder n-Heptyl. Cycloalkyl in obigen Cycloalkanoyl-Gruppen enthält vorzugsweise 3-8, in erster Linie 5 oder 6 Ringglieder und ist z.B. Cyclopentyl oder Cyclohexyl. Phenylniederalkyl in obigen Phenyl-niederalkanoyloxy-Gruppen ist z.B. Benzyl, 1- oder 2-Phenylethyl oder 3-Phenylpropyl. Halogen ist insbesondere Brom, kann jedoch auch für Chlor oder Jod, ferner für Fluor stehen. Niederalkoxy ist z.B. Methoxy, ferner Aethoxy, n-Propoxy, Isopropoxy, n-Butoxy, Isobutoxy oder tert.-Butoxy. Niederalkoxycarbonyl ist z.B. Methoxycarbonyl oder Aethoxycarbonyl, Niederalkoxycarbonyloxy ist z.B. Methoxycarbonyloxy oder Aethoxycarbonyloxy. Heterocyclyl ist z.B. 2-Tetrahydropranyl, 2,3- oder 4-Pyridyl, 2-Thienyl oder 2-Furyl.

Substituenten z.B. von Aromaten sind gegebenenfalls substituiertes Niederalkyl, Niederalkoxy, Nitro und/oder Halogen, oder basische Gruppen, wie eine gegebenenfalls substituierte Aminogruppe, oder saure Gruppen, wie gegebenenfalls funktionell abgewandeltes, z.B. verestertes Carboxy, z.B. Niederalkoxycarbonyl, oder gegebenenfalls veräthertes, verestertes oder geschütztes Hydroxy oder Oxo.

Monocyclisches aromatisches Sulfonyloxy ist z.B. p-Toluolsulfonyloxy oder Benzolsulfonyloxy.

Niederaliphatisches Sulfonyloxy ist z.B. Methansulfonyloxy.

Sofern in einer funktionell abgewandelten Hydroxygruppe salzbildende Substituenten vorhanden sind, können auch Salze der neuen Verbindungen gemäss der Erfindung hergestellt werden, insbesondere pharmazeutisch verwendbare, nicht-toxische Salze. So können z.B. Säureadditionssalze von Estern hergestellt werden, in denen ein gesättigter stickstoffhaltiger Heterocyclylrest vorhanden ist, wie die Salze der Halogenwasserstoffsäuren, Schwefelsäuren oder Phosphorsäuren oder von niederaliphatischen Carbonsäuren. Hemiester von Dicarbonsäuren können Metall-Salze geben, z.B. Alkalimetallsalze die infolge ihrer guten Wasserlöslichkeit von besonderem Interesse sind. Auch die Mono-Ester der Schwefel- oder Phosphorsäuren können in ihre Salze, z.B. in die Alkalisalze übergeführt werden und auch diese Ester beanspruchen deshalb besonderes Interesse.

Zur Isolierung oder Reinigung der neuen Verbindungen können auch pharmazeutisch ungeeignete Salze Verwendung finden.

Das neue durch Fermentation gebildete Antibiotikum, Extrakte der Fermentationsbrühe und insbesondere die Substanzen der Formel (IV) und gegebenenfalls ihre Salze besitzen pharmakologisch wertvolle Eigenschaften. Insbesondere wirken sie antibakteriell und antifungisch

und können daher z.B. in Form pharmazeutischer Präparate, z.B. zur Behandlung von bakteriell oder durch Pilze verursachten Infektionen an Mensch und Tier verwendet werden.

Die antibakterielle Wirkung kommt sowohl gegen gram-positive wie gram-negative Bakterien zur Geltung, wie z.B. im Platten-Agardiffusionstest in vitro [10 µl/Testrondelle (6 mm ⌀)] gezeigt werden kann, wo sie bei verschiedenen pathogenen Keimen eine minimale Hemmkonzentration im Bereiche von 1-200 µg/ml zeigen. Von den gegenüber den neuen Verbindungen empfindlichen gram-positiven Bakterien seien z.B. die folgenden genannt: Bacillus cereus, Bacillus subtilis, Clostridium pasteurianum, Corynebacterium rathayi, von den gram-negativen folgende: Agrobacterium tumefaciens, Erwinia amylovorum, Escherichia coli K 12 wild type, Klebsiella aerogenes, Serratia marcescens 13880, Serratia marcescens 30122.

Die antifungische Wirkung tritt praktisch gegenüber den meisten Pilzen auf. So weist das Antibiotikum Tü 1718 und insbesondere die Verbindungen der Formel (IV) im oben genannten Agardiffusionstest eine minimale Hemmkonzentration im Bereiche von ca. 0,1-200 µg/ml gegenüber sehr vielen pathogenen und nicht pathogenen Pilzen, z.B. gegen Pythium debarianum, Mucor hiemalis+, Mucor hiemalis-, Mucor miehei, Trichophyton mentagrophytes, Botrytis cinerea 157, Aspergillus fumigatus, und mehrere andere. So besitzen z.B. die nachstehend aufgeführten spezifischen Verbindungen die folgenden minimalen Hemmkonzentrationen gegenüber Mucor miehei:

Komponente $A_1$ von Antibiotikum Tü 1718, d.h. (2S,5S)-2-(2-Hydroxyäthyl)-clavam = 10 µg/ml
Phenylurethan von Komponente $A_1$ von Antibiotikum Tü 1718 = 0,1 µg/ml
Phenoxyessigsäureester von Komponente $A_1$ von Antibiotikum Tü 1718 = 0,1 µg/ml.

Das neue Antibiotikum, seine Extrakte und die neuen Verbindungen der Formel (IV) besitzen zudem auch eine tumorhemmende Wirkung, wie in Versuchen an Tumorzellen der Zellinie NS-1, einer Variante der Zelline P 3, die aus Mineralöl-induzierten Plastocytomen der Maus stammen, gezeigt werden kann. So besitzt z.B. das (2S,5S)-2-(2-Hydroxyäthyl)-clavam in diesem Test ca. die Hälfte der tumorhemmenden Wirkung von Actinomycin C.

Das neue Antibiotikum Tü 1718, seine Extrakte und insbesondere die neuen Verbindungen der Formel IV gemäss der Erfindung sind wenig toxisch. So liegen z.B. die lethalen Dosen bei i.p. Gabe an der Maus bei ca. 100-200 mg/kg. Sie können daher innerhalb weiter Dosenbereiche als antibakterielle und insbesondere antifungische Antibiotika in der Human- und Tiermedizin verwendet werden.

Das neue Antibiotikum Tü 1718 bzw. dessen Hauptkomponente, das (2S,5S)-2-(2-Hydroxyäthyl)-clavam, entsteht bei der Züchtung eines neuen Stammes des Mikroorganismus Streptomyces antibioticus, subspec. antibioticus, welcher durch die Bezeichnung Tü 1718 charakterisiert ist, und welcher aus einer Bodenprobe am Flussufer von Karen-Village in Nordthailand isoliert wurde. Der Stamm ist bei der Deutschen Sammlung von Mikroorganismen, Grisebachstrasse 8, Göttingen, am 12. November 1980 hinterlegt worden und wird dort unter der Hinterlegungsnummer DSM 1951 aufbewahrt. Im folgenden wird gelegentlich für diesen Stamm die abgekürzte Schreibweise Str.a. Tü 1718 verwendet. Der Stamm ist durch folgende Merkmale charakterisiert:

Nach der Bestimmung und Nomenklatur von Hütter (1967) ergeben sich folgende Kennzeichen:

| | |
|---|---|
| Sporengrösse: | 0,5-1 µm breit, 1,2-2 µm lang |
| Sporenform: | ellipsoid bis länglich |
| Sporenoberfläche: | glatt bis warzig |
| Sporenmorphologie: | monopodial verzweigt, gerade oder gewellt (RF) |
| Chromogenität: | positiv auf peptonhaltigen Nährböden |
| Luftmycelfarbe: | anfangs kreideweiss, später grauweiss und schliesslich grau, cinereus. |

**Durch diese Merkmale charakterisiert kann der Stamm als Streptomyces antibioticus bestimmt werden. Sporenketten dieses Stammes sind in Fig. 1 wiedergegeben.**

Sporenketten von Streptomyces antibioticus Tü 1718

Nach Bergey's Manual of Determinative Bacteriology (1975) erhält der Stamm folgende Kennzeichen: Gy, RF, C+, SM.

Weiterhin wird die Beurteilung des Wachstums auf verschiedenen C-Quellen aufgeführt. Die folgende Tabelle 1 enthält die Ergebnisse von Bergey's Manual, Nonomura (1974) und Pridham & Gottlieb (1948) im Vergleich zu Str.a. Tü 1718.

Tabl. 1: C-Quellen-Verwertung von Tü 1718

| | Kontrolle | D-Glucose | D-Xylose | L-Arabinose | L-Rhamnose | D-Fructose | D-Galactose | Raffinose | D-Mannit | Inosit |
|---|---|---|---|---|---|---|---|---|---|---|
| Str.a. Tü 1718 | - | + | ± | + | + | + | + | ± | + | + |
| Streptomyces antibiot. Bergey's Manual | - | + | + | + | + | + | + | - | + | + |
| Streptomyces antibiot. Nonomura, 1974 | - | o | ± | + | + | + | o | - | + | + |
| Streptomyces antibiot. Pridham et al., 1948 | - | o | + | + | + | + | + | - | + | + |

Wachstum: + gut

± indifferent

- kein

o nicht getestet.

Danach stimmt Str.a. Tü 1718 recht gut im C-Quellen-Spektrum mit Streptomyces antibioticus überein und erhält den Namen Streptomyces antibioticus ssp. antibioticus. Str. a. Tü 1718 tritt spontan in einer weissen Variante auf, die durch langsamere Antibiotika-Produktion gekennzeichnet ist. Weiterhin kann bei der Mutation mit N-Methyl-N'-nitro-N-nitrosoguanidin eine grüne Variante isoliert werden, die genauso viel produziert wie der Wildtyp.

Es sind somit spontan oder künstlich in an sich bekannter Weise zu erzeugende Mutanten zugänglich, die ebenso wie der Wildstamm die neue oben genannte Clavam-Verbindung gemäss der vorliegenden Erfindung zu

erzeugen vermögen. Solche Mutanten können durch chemische Mittel, wie das oben bereits genannte Guanidinderivat, oder z.B. auch mit Senfölen oder durch Bestrahlung, z.B. mit Ultraviolett- oder Röntgenstrahlen, erhalten werden.

Das Verfahren der vorliegenden Erfindung zur Herstellung des Antibiotikums Tü 1718 und/oder der neuen Clavam-Verbindungen der Formel (IV) ist somit dadurch gekennzeichnet, dass man den Stamm Tü 1718 der Art Streptomyces antibioticus ssp. antibioticus oder eine sich von diesem Stamm ableitende, das Antibiotikum Tü 1718 produzierende Mutante, in einem eine Kohlenstoff- und Stickstoffquelle und anorganische Salze enthaltenden flüssigen Nährmedium aerob züchtet, bis die Nährlösung die gewünschte antibiotische Wirkung zeigt, wenn erwünscht, das entstandene Antibiotikum und/oder das darin enthaltene (2S,5S)-2-(2-Hydroxyäthyl)-clavam isoliert und, wenn erwünscht, dieses in seine an der Hydroxygruppe funktionell abgewandelten Derivate, und diese gegebenenfalls in ihre Salze überführt.

Die zur Züchtung nötige Nährlösung muss eine Kohlenstoff- und Stickstoffquelle sowie anorganische Salze enthalten. Als Kohlenstoffquelle sind beispielsweise zu nennen: assimilierbare Kohlenhydrate, z.B. die oben bereits in der Tabelle angeführten, insbesondere D-Mannit oder Lactose.Als stickstoffhaltige Nährstoffe seien genannt:Aminosäuren,Peptide und Proteine sowie deren Abbauprodukte wie Pepton oder Trypton, ferner Fleischextrakte, Getreidemehle, z.B. von Mais, Weizen, Bohnen, insbesondere der Sojapflanze, von Samen, beispielsweise der Baumwollpflanze, Destillationsrückstände der Alkoholherstellung, Hefeextrakte usw., aber auch Ammoniumsalze und Nitrate. Von anderen anorganischen Salzen kann die Nährlösung beispielsweise Chloride, Carbonate, Sulfate, Phosphate von Alkali- oder Erdalkalimetallen, von Magnesium, Eisen, Zink und Mangan enthalten.

Besonders gut verläuft die Bildung des Antibiotikums in einer Nährlösung, die ca. 2% Mannit und 2% Sojamehl enthält.

Die Züchtung erfolgt aerob, also beispielsweise submers unter Schütteln oder Rühren mit Luft oder Sauerstoff in Schüttelkolben oder den bekannten Fermentern. Als Temperatur eignet sich eine solche zwischen 18 und 40°C, vorzugweise ca. 27-28°C. Eine wesentliche antibiotische Wirkung zeigt die Nährlösung dabei im allgemeinen nach 2-3 Tagen. Vorzugsweise kultiviert man in mehreren Stufen, d.h. man stellt zunächst eine oder mehrere Vorkulturen in flüssigem Nährmedium her, die dann in das eigentliche Produktionsmedium der Hauptkultur, z.B. im Verhältnis 1:10, überimpft werden. Die Vorkultur erhält man beispielsweise, indem man ein durch ca. 14-tägiges Wachstum auf einem festen oder flüssigen Nährboden, z.B. Hefe-Malz-Agar, enthaltenes versportes Mycel in eine Nährlösung überimpft und 30-60, z.B. 45 Stunden, wachsen lässt.

Der Fermentationsverlauf kann anhand folgender Kriterien überwacht werden: pH-Wert der Kultur, Nassvolumen, 10 ml Kulturbrühe werden 5 Minuten bei 2000 g zentrifugiert und die Menge des abzentrifugierten Sediments ermittelt; Dünnschichtchromatographie; biologische Aktivität.

Die Isolierung des Antibiotikums und/oder seiner Hauptkomponente aus dem Kulturmedium erfolgt nach an sich bekannten Methoden unter Berücksichtigung der chemischen, physikalischen und biologischen Eigenschaften der Substanzen. Zum Testieren der Antibiotika-Konzentration in den einzelnen Isolierungsstufen - wie auch im Kulturmedium - kann die Dünnschichtchromatographie z.B. auf Silicagel (z.B. mit Chloroform-Methanol) und/oder die Bioautographie mit verschiedenen Mikroorganismen verwendet werden. Die Rf-Werte der Hauptkomponente (2S,5S)-2-(2-Hydroxyäthyl)-clavam in verschiedenen Laufmittelsystemen sind in einem der nachfolgenden Illustrationsbeispiele zu finden. Die Menge des gebildeten Antibiotikums wird mit einer Eichlösung verglichen.

Bei der Autobiographie kann der Plattendiffusionstest dienen, wobei vorzugsweise folgende Testkeime verwendet werden: Clostridium pasteurianum, Escherichia coli und Mucor miehei. Die minimale Hemmkonzentration der Hauptkomponente des Antibiotikums in µg/ml gegenüber den eben genannten Mikroorganismen ist wie folgt:

Clostridium pasteurianum 50

Mucor miehei 10-20.

Sodann kann für die Identifizierung des Antibiotikums auf der Dünnschichtchromatographie-Platte auch das Besprühen mit Ehrlich's Reagenz (Rotfärbung) oder mit Vanillin/$H_2SO_4$ (Grünfärbung) dienen.

Das Antibiotikum kann aus der unfiltrierten Kulturbrühe mit einem mit Wasser nicht oder wenig mischbaren organischen Lösungsmittel, z.B. Chloroform, extrahiert werden. Dieses sogenannte "whole broth"-Verfahren ist indessen weniger zu empfehlen, weil sich das Antibiotikum nur zum geringsten Teil im Mycel befindet. Es ist daher vorteilhaft, die Kulturbrühe vom Mycel, z.B. durch Filtration oder durch Zentrifugation, vorzugsweise unter Zugabe von Celite, zu trennen, und es aus dem Filtrat zu isolieren.

Man kann das Antibiotikum aus dem Filtrat mit einem mit Wasser wenig oder nicht mischbaren Lösungsmittel, wie insbesondere einem chlorierten Kohlenwasserstoff, z.B. Chloroform, Methylenchlorid oder Aethylenchlorid, oder Essigester, extrahieren, jedoch ist es vorteilhaft, zuerst das Antibiotikum an Aktivkohle oder an einem makroretikulären Harz ohne funktionelle Gruppen, wie z.B. XAD-2, DIAION HP-20 und ähnliche, zu adsorbieren, es daraus z.B. mit Aceton zu eluieren, und es erst dann mit einem der genannten Lösungsmittel zu extrahieren. So behandelt man vorteilhaft das wie oben beschrieben vom Mycel getrennte Filtrat der Kulturbrühe mit 5%iger granulierter Aktivkohle ca. 2 Stunden lang, presst dann ab und extrahiert die Kohle für ca. 1 Stunde mit 10% des Fermentationsvolumens Aceton oder ähnlichen höheren aliphatischen Ketonen. Der Keton-Extrakt wird bis zum wässrigen Rückstand (ca. 1% des Fermentationsvolumens) eingedampft.

Das so vorgereinigte Antibiotikum enthält drei bis jetzt isolierte Komponenten: die Hauptkomponente $A_1$, das oben beschriebene (2S,5S)-2-(2-Hydroxyäthyl)-clavam, und zwei Nebenkomponenten $A_2$ und B, von denen die erste ein bisher nocht nicht identifiziertes Antibiotikum und die andere ein valinhaltiges Dipeptidderivat ist.

Zur Isolierung und Reinigung der Hauptkomponente ($A_1$) wird der oben erhaltene Aceton- bzw. Keton-Extrakt mit einem chlorierten Kohlenwasserstoff, wie insbesondere einem der oben schon erwähnten, insbesondere Chloroform, extrahiert. Man extrahiert z.B. 5mal mit 0,5% des Fermentationsvolumens Chloroform und engt dann die vereinigten Extrakte auf ein kleines Volumen ein und reinigt diese Substanz weiter mittels präparativer Dünnschichtchromatographie oder Chromatographie an einer Kieselgel-Säule, wobei als Fliessmittel vorzugsweise Gemische von Chloroform/Methanol 92:8 verwendet werden.

Aus dem Rückstand des Aceton-Extraktes, nachdem die Hauptkomponente mit Chloroform entfernt ist, können durch verschiedene Reinigungsverfahren die zwei Nebenkomponenten $A_2$ und B erhalten werden.

Zur vollständigen Reinigung der Hauptkomponente unterwirft man den Chloroform-Extrakt einer oder mehreren der folgenden Operationen:

a) Chromatographie an Kieselgel mit Chloroform/Methanol 9:1
b) Chromatographie an Polyamid mit Chloroform
c) Gel-Filtration an Sephadex LH 20 mit Chloroform
d) Craig-Verteilung im System n-Hexan, iso-Propyläther, Aceton, 0,1 M Phosphatpuffer pH 7 (8:25:25:25).

Die Substanz ist nach einer Kombination der Reinigungsschritte a, b und c oder a und d rein.

Die Hauptkomponente des Antibiotikums kann selbstverständlich auch

 

nach anderen Varianten dieser an sich bekannten Reinigungsmethoden, z.B. mit anderen Lösungsmitteln bzw. Lösungsmittelgemischen und anderen Absorptionsmitteln erhalten werden.

Das reine (2S,5S)-2-(2-Hydroxyäthyl)-clavam stellt eine farblose ölige Substanz dar, die in Chloroform, Aceton, Methanol sehr gut und in Wasser weniger gut löslich ist. Die Substanz ist in lösungsmittelfreiem Zustand beschränkt stabil und wandelt sich in eine in jedem Lösungsmittel unlösliche Substanz um. Als Aufbewahrungsmittel eignet sich Chloroform. In wässerigen Lösungen ist eine einigermassen gute Stabilität bei pH 5-7 gegeben. Es ist somit möglich, die Verbindungen in Form all jener pharmazeutischen Präparate zu verwenden, die Wasser enthalten, wie insbesondere von wässrigen Lösungen bei der parenteralen oder oralen Applikation, oder Cremen oder Salben oder Tinkturen bei der topischen Gabe.

Die Hydroxygruppe des (2S,5S)-2-(2-Hydroxyäthyl)-clavams kann in an sich bekannter Weise verestert oder veräthert werden. Geeignete Mittel zur Verätherung sind beispielsweise Diazoverbindungen, wie gegebenenfalls substituierte Diazoniederalkane, z.B. Diazomethan, Diazoäthan, Diazo-n-butan oder Diphenyldiazomethan. Diese Reagenzien werden in Gegenwart eines geeigneten inerten Lösungsmittels, wie eines aliphatischen, cycloaliphatischen oder aromatischen Kohlenwasserstoffs, wie Hexan, Cyclohexan, Benzol oder Toluol, eines halogenierten aliphatischen Kohlenwasserstoffes, z.B. Methylenchlorids, oder eines Aethers, wie eines Diniederalkyläthers, z.B. Diäthyläther, oder eines cyclischen Aethers, z.B. Tetrahydrofuran oder Dioxan, oder eines Lösungsmittelgemisches, angewendet.

Weitere geeignete Verätherungsmittel sind Ester entsprechender Alkohole, in erster Linie solche mit starken anorganischen oder organischen Säuren, wie Mineralsäuren, z.B. Halogenwasserstoffsäuren, wie Chlorwasserstoff-, Bromwasserstoff- oder Jodwasserstoffsäure,ferner

Schwefelsäure,oder Halogen-schwefelsäuren, z.B. Fluorschwefelsäure, oder starken organischen Sulfonsäuren, wie gegebenenfalls, z.B. durch Halogen, wie Fluor, substituierten Niederalkansulfonsäuren, oder aromatischen Sulfonsäuren, wie z.B. gegebenenfalls, durch Niederalkyl, wie Methyl, Halogen, wie Brom, und/oder Nitro substituierten Benzolsulfonsäuren, z.B. Methansulfon-, Trifluormethansulfon- oder p-Toluolsulfonsäure. Solche Ester sind u.a. Niederalkylhalogenide, Diniederalkylsulfate, wie Dimethylsulfat, ferner Fluorsulfonsäureester, wie -niederalkylester,z.B. Fluorsulfonsäuremethylester, oder gegebenenfalls Halogen-substituierte Methansulfonsäure-niederalkylester, z.B. Trifluormethansulfonsäuremethylester. Sie werden üblicherweise in Gegenwart eines inerten Lösungsmittels, wie eines gegebenenfalls halogenierten, wie chlorierten, aliphatischen, cycloaliphatischen oder aromatischen Kohlenwasserstoffs, z.B. Methylenchlorid, eines Aethers, wie Dioxan oder Tetrahydrofuran, oder eines Gemisches verwendet. Dabei wendet man vorzugsweise geeignete Kondensationsmittel, wie Alkalimetallcarbonate oder -hydrogencarbonate, z.B. Natrium- oder Kaliumcarbonat oder -hydrogencarbonat (üblicherweise zusammen mit einem Sulfat), oder organische Basen, wie, üblicherweise sterische gehinderte, Triniederalkylamine, z.B. N,N-Diisopropyl-N-äthyl-amin (vorzugsweise zusammen mit Halogensulfonsäure-niederalkylestern oder gegebenenfalls Halogensubstituierten Methansulfonsäure-niederalkylestern) an, wobei unter Kühlen, bei Raumtemperatur oder unter Erwärmen, z.B. bei Temperaturen von etwa -20°C bis etwa 50°C und, wenn notwendig, in einem geschlossenen Gefäss und/oder in einer Inertgas-, z.B. Stickstoffatmosphäre gearbeitet wird.

Durch Phasentransfer-Katalyse [siehe Dehmlow, Angewandte Chemie, Bd. 5, S. 187 (1974)] kann die oben beschriebene Verätherungsreaktion wesentlich beschleunigt werden. Als Phasentransfer-Katalysatoren können quartäre Phosphoniumsalze und insbesondere quartäre Ammoniumsalze, wie gegebenenfalls substituierte Tetraalkylammoniumhalogenide, z.B. Tetrabutylammoniumchlorid, -bromid oder -jodid,

oder auch Benzyl-triäthylammoniumchlorid, in katalytischen oder bis zu äquimolaren Mengen verwendet werden. Als organische Phase kann irgendein der mit Wasser nicht mischbaren Lösungsmittel dienen, beispielsweise einer der gegebenenfalls halogenierten, wie chlorierten niederaliphatischen, cycloaliphatischen oder aromatischen Kohlenwasserstoffe, wie Tri- oder Tetrachloräthylen, Tetrachloräthan, Tetrachlorkohlenstoff, Chlorbenzol, Toluol oder Xylol. Die als Kondensationsmittel geeigneten Alkalimetallcarbonate oder -hydrogencarbonate, z.B. Kalium- oder Natriumcarbonat oder -hydrogencarbonat, Alkalimetallphosphate, z.B. Kaliumphosphat, und Alkalimetallhydroxide, z.B. Natriumhydroxid, können bei basenempfindlichen Verbindungen der Reaktionsmischung titrimetrisch, z.B. mittels eines Titrierautomaten, zugesetzt werden, damit der pH-Wert während der Verätherung zwischen etwa 7 und etwa 8.5 bleibt.

Weitere Verätherungsmittel sind geeignete Acetal-Verbindungen, z.B. gem-Di-niederalkoxy-niederalkane, wie 2,2-Dimethoxy-propan, die in Gegenwart von starken organischen Sulfonsäuren, wie p-Toluolsulfonsäure, und eines geeigneten Lösungsmittel, wie eines Diniederalkyl- oder Niederalkylensulfoxyds, z.B. Dimethylsulfoxyd, zur Anwendung gelangen, oder geeignete Orthoester, z.B. Orthoameisensäuretriniederalkylester, z.B. Orthoameisensäure-triäthylester, die in Gegenwart einer starken Mineralsäure, z.B. Schwefelsäure, oder einer starken organischen Sulfonsäure, wie p-Toluolsulfonsäure, und eines geeigneten Lösungsmittels, wie eines Aethers, z.B. Dioxan, verwendet werden.

Weitere Verätherungsmittel sind entsprechende trisubstituierte Oxoniumsalze (sogenannte Meerweinsalze), oder disubstituierte Carbenium- oder Haloniumsalze, worin die Substituenten die veräthernden Reste sind, beispielsweise Triniederalkyloxoniumsalze, sowie Diniederalkoxycarbenium- oder Diniederalkylhaloniumsalze, insbesondere die entsprechenden Salze mit komplexen, fluorhaltigen Säuren, wie die entsprechenden Tetrafluorborate, Hexafluorphosphate, Hexafluorantimonate oder Hexachlorantimonate. Solche Reagentien sind z.B. Trimethyloxonium- oder Triäthyloxonium-hexafluorantimonat, -hexachlorantimonat, -hexafluorphosphat oder -tetrafluorborat, Dimethoxycarbeniumhexafluorphosphat oder Dimethylbromoniumhexafluorantimonat. Man verwendet diese Verätherungsmittel vorzugsweise in einem inerten Lösungsmittel, wie einem Aether oder einem halogenierten Kohlenwasserstoff, z.B. Diäthyläther, Tetrahydrofuran oder Methylenchlorid, oder in einem Gemisch davon, wenn notwendig, in Gegenwart einer Base, wie einer organischen Base, z.B. eines, vorzugsweise sterisch gehinderten, Triniederalkylamins, z.B. N,N-Diisopropyl-N-äthyl-amin, und unter Kühlen, bei Raumtemperatur oder unter leichtem Erwärmen, z.B. bei etwa -20°C bis etwa 50°C, wenn notwendig, in einem geschlossenen Gefäss und/oder in einer Inertgas-, z.B. Stickstoffatmosphäre.

Weitere Verätherungsmittel sind schliesslich entsprechende 1-substituierte 3-Aryltriazenverbindungen, worin der Substituent den veräthernden Rest, und Aryl vorzugsweise gegebenenfalls substituiertes Phenyl, z.B. Niederalkylphenyl, wie 4-Methylphenyl, bedeutet. Solche Triazenverbindungen sind 3-Aryl-1-niederalkyltriazene, z.B. 3-(4-Methylphenyl)-1-methyl-triazen, 3-(4-Methylphenyl)-1-äthyl-triazen oder 3-(4-methylphenyl)-1-isopropyl-triazen. Diese Reagentien werden üblicherweise in Gegenwart von inerten Lösungsmitteln, wie gegebenenfalls halogenierten Kohlenwasserstoffen oder Aethern, z.B. Benzol, oder Lösungsmittelgemischen, und unter Kühlen, bei Raumtemperatur und vorzugsweise bei erhöhter Temperatur, z.B. bei etwa 20°C bis etwa 100°C, wenn notwendig, in einem geschlossenen Gefäss und/oder in einer Inertgas-, z.B. Stickstoffatmosphäre verwendet.

Zur Veresterung der Hydroxygruppe behandelt man das Ausgangsmaterial der Formel (IV) mit einem den gewünschten Acylrest einer organischen Carbonsäure einführenden Acylierungsmittel. Dabei verwendet man die entsprechende Carbonsäure oder ein reaktionsfähiges Derivat davon,

insbesondere ein Anhydrid, inkl. ein gemischtes oder inneres Anhydrid einer solchen Säure. Gemischte Anhydride sind z.B. diejenigen mit Halogenwasserstoffsäuren, d.h. die entsprechenden Säurehalogenide, insbesondere -chloride, ferner mit Cyanwasserstoffsäure, oder dann diejenigen mit geeigneten Kohlensäurehalbderivaten, wie entsprechenden -halbestern (wie die z.B. mit einem Halogen-ameisensäure-niederalkyl, wie Chlorameisensäure-äthylester oder -isobutylester, gebildeten gemischten Anhydride) oder mit gegebenenfalls substituierten, z.B. Halogen, wie Chlor, enthaltenden Niederalkancarbonsäuren (wie die mit Pivalinsäurechlorid oder Trichloressigsäurechlorid gebildeten gemischten Anhydride). Innere Anhydride sind z.B. diejenigen von organischen Carbonsäuren, d.h. Ketene, wie Keten oder Diketen, oder diejenigen von Carbamin- oder Thiocarbaminsäuren, d.h. Isocyanate oder Isothiocyanate. Weitere reaktionsfähige, als Acylierungsmittel verwendbare Derivate von organischen Carbonsäuren sind aktivierte Ester, wie geeignet substituierte Niederalkylester, z.B. Cyanmethylester, oder geeignet substituierte Phenylester, z.B. Pentachlorphenyl- oder 4-Nitrophenylester. Die Veresterung kann, wenn notwendig, in Gegenwart von geeigneten Kondensationsmitteln, bei Verwendung von freien Carbonsäuren, z.B. in Gegenwart von Carbodiimidverbindungen, wie Dicyclohexylcarbodiimid, oder Carbonylverbindungen, wie Diimidazolylcarbonyl, und bei Verwendung von reaktionsfähigen Säurederivaten z.B. in Gegenwart von basischen Mitteln, wie Triniederalkylaminen, z.B. Triäthylamin, oder heterocyclischen Basen, z.B. Pyridin oder 4-Dimethylaminopyridin, durchgeführt werden. Die Acylierungsreaktion kann in Abwesenheit oder in Gegenwart eines Lösungsmittels oder Lösungsmittelgemisches, unter Kühlen, bei Raumtemperatur oder unter Erwärmen, und, wenn notwendig, in einem geschlossenen Gefäss und/oder in einer Inertgas-, z.B. Stickstoffatomosphäre, durchgeführt werden. Geeignete Lösungsmittel sind z.B. gegebenenfalls substituierte, insbesondere gegebenenfalls chlorierte, aliphatische, cycloaliphatische oder aromatische Kohlenwasserstoffe, wie Benzol oder Toluol, wobei man geeignete Veresterungsreagentien, wie Essigsäureanhydrid, auch als Verdünnungsmittel verwenden kann.

Eine durch eine organische Sulfonsäure, z.B. Niederalkansulfonsäure, wie Methansulfonsäure, oder eine aromatische Sulfonsäure, wie p-Toluolsulfonsäure, veresterte Hydroxygruppe kann man vorzugsweise durch Behandeln des Ausgangsmaterials der Formel (I) mit einem reaktionsfähigen Sulfonsäurederivat, wie einem entsprechenden Halogenid, z.B. Chlorid, wenn notwendig, in Gegenwart eines Säure-neutralisierenden basischen Mittels, z.B. einer anorganischen oder organischen Base, z.B. in analoger Weise wie die entsprechenden Ester mit organischen Carbonsäuren, bilden.

Beim Umsetzen eines Salzes einer Thiocarbonsäure, z.B. einer der früher genannten, mit einem Sulfonsäureester oder einem anderen, eine Elektronen-anziehende Gruppe enthaltenden Ester, wie das Dichloracetat, des (2S,5S)-2-(2-Hydroxyäthyl-clavams gemäss der Erfindung erhält man die entsprechenden Thioester dieser Verbindung. Die Reaktion kann in an sich bekannter Weise, z.B. durch Erwärmen mit der doppelten Menge eines Alkalimetallsalzes der gewünschten Thiosäure, z.B. mit Kaliumthioacetat, ausgeführt werden. Als Sulfonsäureester benutzt man vorzugsweise einen von einer niederaliphatischen Sulfonsäure, wie Methansulfonsäure, oder von einer monocyclischen aromatischen Sulfonsäure, wie p-Toluolsulfonsäure, sich ableitenden Ester. Auch andere bekannte Methoden zur Darstellung von Thiocarbonsäureester, die nicht zu einer sonstigen Veränderung des Clavam-Ringsystems führen, können verwendet werden, z.B. durch Umesterung eines Carbonsäureesters mit einem grossen Ueberschuss an Thiosäure.

Zur Herstellung von Thioäthern des erfindungsgemässen Antibiotikums kann man ebenfalls die Sulfonsäuren als Ausgangsstoffe verwenden, z.B. die oben spezifisch genannten, und sie mit einem Alkalisalz des gewünschten Mercaptans umsetzen. Zu diesem Zwecke setzt man z.B. das in etwa 15-20%iger Natronlauge gelöste Mercaptan mit einem kleinen Ueberschuss der antibiotischen Substanz bei niederer Temperatur um.

Enthält die Estergruppe eine salzbildende Gruppe, wie z.B. im Falle, dass die Estergruppe sich von einer mehrbasischen Carbonsäure oder von einer anorganischen Säure, wie einer Schwefel- oder Phosphorsäure ableitet, so können die entsprechenden Ester in Metallsalze, insbesondere in die Alkalimetallsalze wie das Kalium- oder Natriumsalz oder in die Salze von organischen Basen, vorzugsweise therapeutisch verwendbaren Stickstoffbasen wie z.B. Aethanolamin, Diäthanolamin, Triäthanolamin, Dibenzyläthylendiamin, Ephedrin oder 1-Phenyl-2-methyl-3-aminopropan übergeführt werden. Solche Salze sind in Wasser sehr leicht löslich und daher besonders zur Herstellung von parenteral zu verwendenden antibiotischen Präparaten geeignet. Infolge der engen Verwandtschaft zwischen den Salzen und den oben besprochenen Clavamderivaten gemäss der Erfindung gelten die obigen Ausführungen betreffend diese Derivate sinngemäss auch für die Salze, die ebenso zum Gegenstand der Erfindung gehören.

Die Salze können in an sich bekannter Weise hergestellt werden, z.B. durch Umsetzen der freien Verbindungen mit einem Alkali- oder Erdalkalimetallhydroxid, -carbonat, -hydrogencarbonat, -amid oder -hydrid oder mit einem geeigneten Alkalimetall-niederalkanoat, oder mit Ammoniak oder einem Amin, wie einem der oben angeführten. Man stellt so insbesondere pharmazeutisch anwendbare Salze her.

Die Salze können aus ihren Lösungen z.B. durch Lyophilisation in eine für die pharmazeutische Verwendung geeignete Form gebracht werden.

Die Erfindung betrifft auch diejenigen Ausführungsformen des obigen Verfahrens, bei denen man von einer auf irgendeiner Stufe als Zwischenprodukt erhältlichen Verbindung ausgeht und die fehlenden Schritte durchführt oder man das Verfahren auf irgendeiner Stufe abbricht oder man eine nach dem erfindungsgemässen Verfahren erhältliche Verbindung unter den Verfahrensbedingungen erzeugt und in situ weiterverarbeitet.

Die vorliegende Erfindung betrifft ebenfalls pharmazeutische Präparate, welche eine Verbindung gemäss der Formel IV oder gegebenenfalls ein Salz davon als Wirksubstanz enthalten, sowie Verfahren zu deren Herstellung. Bei den erfindungsgemässen pharmazeutischen Präparaten handelt es sich um solche zur oralen oder rektalen oder parenteralen und insbesondere topischen Verabreichung, welche den pharmakologischen Wirkstoff allein oder zusammen mit einem entsprechenden pharmazeutisch anwendbaren Trägermaterial enthalten.

Als topisch anwendbare pharmazeutische Präparate kommen z.B. Crèmen, Salben, Pasten, Schäume, Tinkturen und Lösungen in Betracht, die von etwa 0,02% bis etwa 2% des Wirkstoffes enthalten.

Crèmen sind Oel-in-Wasser-Emulsionen, die mehr als 50% Wasser aufweisen. Als ölige Grundlage verwendet man in erster Linie Fettalkohole, z.B. Lauryl-, Cetyl- oder Stearylalkohol, Fettsäuren, z.B. Palmitin- oder Stearinsäure, flüssige bis feste Wachse, z.B. Isopropylmyristat, Wollwachs oder Bienenwachs, und/oder Kohlenwasserstoffe, z.B. Vaseline (Petrolatum) oder Paraffinöl. Als Emulgatoren kommen oberflächenaktive Substanzen mit vorwiegend hydrophilen Eigenschaften in Frage, wie entsprechende nichtionische Emulgatoren, z.B. Fettsäureester von Polyalkoholen oder Aethylenoxidaddukte davon, wie Polyglycerinfettsäureester oder Polyoxyäthylensorbitan-fettsäureester (Tweens), ferner Polyoxyäthylen-fett-alkoholäther oder -fettsäureester, oder entsprechende ionische Emulgatoren, wie Alkalimetallsalze von Fettalkoholsulfaten, z.B. Natriumlaurylsulfat, Natriumcetylsulfat oder Natriumstearylsulfat, die man üblicherweise in Gegenwart von Fettalkoholen, z.B. Cetylalkohol oder Stearylalkohol, verwendet. Zusätze zur Wasserphase sind u.a. Mittel, welche die Austrocknung der Crèmen vermindern, z.B. Polyalkohole, wie Glycerin, Sorbit, Propylenglykol und/oder Polyäthylenglykole, ferner Konservierungsmittel, Riechstoffe etc.

Salben sind Wasser-in-Oel-Emulsionen, die bis zu 70%, vorzugsweise jedoch von etwa 20% bis etwa 50% Wasser oder wässerige Phase enthalten. Als Fettphase kommen in erster Linie Kohlenwasserstoffe, z.B. Vaseline, Paraffinöl und/oder Hartparaffine in Frage, die zur Verbesserung des Wasserbindungsvermögens vorzugsweise geeignete Hydroxyverbindungen, wie Fettalkohole oder Ester davon, z.B. Cetylalkohol oder Wollwachsalkohole, bzw. Wollwachs, enthalten. Emulgatoren sind entsprechende lipophile Substanzen, wie Sorbitan-fettsäureester (Spans), z.B. Sorbitanoleat und/oder Sorbitanisostearat. Zusätze zur Wasserphase sind u.a. Feuchthaltungsmittel, wie Polyalkohole, z.B. Glycerin, Propylenglykol, Sorbit und/oder Polyäthylenglykol, sowie Konservierungsmittel, Riechstoffe, etc.

Fettsalben sind wasserfrei und enthalten als Grundlage insbesondere Kohlenwasserstoffe, z.B. Paraffin, Vaseline und/oder flüssige Paraffine, ferner natürliche oder partialsynthetische Fette, z.B. Kokosfettsäuretriglycerid, oder vorzugsweise gehärtete Oele, z.B. hydriertes Erdnuss- oder Rizinusöl, ferner Fettsäurepartialester des Glycerins, z.B. Glycerinmono- und -distearat, sowie z.B. die im Zusammenhang mit den Salben erwähnten, die Wasseraufnahmefähigkeit steigernden Fettalkohole, Emulgatoren und/oder Zusätze.

Pasten sind Crèmen und Salben mit sekretabsorbierenden Puderbestandteilen, wie Metalloxiden, z.B. Titanoxid oder Zinkoxid, ferner Talk und/oder Aluminiumsilikate, welche die Aufgabe haben, vorhandene Feuchtigkeit oder Sekrete zu binden.

Schäume werden aus Druckbehältern verabreicht und sind in Aerosolform vorliegende flüssige Oel-in-Wasser-Emulsionen, wobei halogenierte Kohlenwasserstoffe, wie Chlorfluorniederalkane, z.B. Dichlordifluormethan und Dichlortetrafluoräthan, als Treibmittel verwendet werden. Als Oelphase verwendet man u.a. Kohlenwasserstoffe, z.B. Paraffinöl, Fettalkohole, z.B. Cetylalkohol, Fettsäureester, z.B. Isopropylmyristat, und/oder andere Wachse. Als Emulgatoren verwendet man u.a. Gemische von solchen mit vorwiegend hydrophilen Eigenschaften, wie Polyoxyäthylen-sorbitan-fettsäureester (Tweens), und solchen mit vorwiegend lipophilen Eigenschaften, wie Sorbitanfettsäureester (Spans). Dazu kommen die üblichen Zusätze, wie Konservierungsmittel, etc.

Tinkturen und Lösungen weisen meistens eine wässerig-äthanolische Grundlage auf, der u.a. Polyalkohole, z.B. Glycerin, Glykole, und/oder Polyäthylenglykol, als Feuchthaltemittel zur Herabsetzung der Verdunstung, und rückfettende Substanzen, wie Fettsäureester mit niedrigen Polyäthylenglykolen, d.h. im wässrigen Gemisch lösliche, lipophile Substanzen als Ersatz für die der Haut mit dem Aethanol entzogenen Fettsubstanzen, und, falls notwendig, andere Hilfs- und Zusatzmittel beigegeben sind.

Die Herstellung der topisch verwendbaren pharmazeutischen Präparate erfolgt in an sich bekannter Weise, z.B. durch Lösen oder Suspendieren des Wirkstoffs in der Grundlage oder in einem Teil davon, falls notwendig. Bei Verarbeitung des Wirkstoffs als Lösung wird dieser in der Regel vor der Emulgierung in einer der beiden Phasen gelöst; bei Verarbeitung als Suspension wird er nach der Emulgierung mit einem Teil der Grundlage vermischt und dann dem Rest der Formulierung beigegeben.

Ausser den topisch verabreichbaren pharmazeutischen Präparaten kommen auch solche zur enteralen, z.B. oralen, sowie parenteralen Verabreichung an Warmblüter in Frage, welche den pharmakologischen Wirkstoff allein oder zusammen mit einem pharmazeutisch anwendbaren Trägermaterial enthalten. Diese pharmazeutischen Präparate enthalten von etwa 10% bis etwa 90% des Wirkstoffs, insbesondere 20-75%, und sind Präparate in Dosiseinheitsform, wie Dragées, Tabletten, Kapseln, Suppo-

sitorien oder Ampullen. Sie werden in an sich bekannter Weise, z.B. mittels konventioneller Misch-, Granulier-, Dragier-, Lösungs- oder Lyophilisierungsverfahren hergestellt.

Geeignete Trägerstoffe für Tabletten und/oder Dragées sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister unter Verwendung z.B. von Mais-, Weizen-, Reis- oder Kartoffelstärke, Gelatine, Tragacanth, Methylcellulose, Hydroxypropylmethylcellulose, Natriumcarboxymethylcellulose und/oder Polyvinylpyrrolidon, und/oder, wenn erwünscht, Sprengmittel, wie die obgenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyäthylenglykol. Dragée-Kerne werden mit geeigneten, gegebenenfalls Magensaft-resistenten Ueberzügen versehen, wobei man u.a. konzentrierte Zuckerlösungen, welche gegebenenfalls arabischen Gummi, Talk, Polyvinylpyrrolidon, Polyäthylenglykol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffen beigefügt werden.

Weitere, oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit Füllstoffen, wie Lactose, Bindemitteln, wie Stärken, und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Oelen, Paraffinöl oder flüssigen Polyäthylenglykolen, gelöst oder suspendiert, wobei ebenfalls Stabilisatoren zugefügt sein können.

Zur parenteralen Verabreichung eignen sich in erster Linie wässrige Lösungen eines Wirkstoffs in wasserlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Oele, z.B. Sesamöl, oder synthetische Fettsäureester, z.B. Aethyloleat oder Triglyceride, verwendet, oder wässrige Injektionssuspensionen, welche viskositätserhöhende Stoffe, z.B. Natrium-Carboxymethylcellulose, Sorbit und/oder Dextran und gegebenenfalls auch Stabilisatoren enthalten.

Die neuen Antibiotika können insbesondere auch in der Tiermedizin verwendet werden.

Die Erfindung umfasst ebenfalls die Verwendung der neuen Antibiotika oder deren pharmazeutisch verwertbaren Salze als pharmakologisch wirksame Stoffe, insbesondere als antifungische Mittel, vorzugsweise in Form von pharmazeutischen Präparaten. Die Dosierung des Wirkstoffes hängt von der Warmblüter-Spezies, dem Körpergewicht und Alter und vom individuellen Zustand, sowie von der Applikationsart ab. Durchschnittlich wird einem Warmblüter von etwa 70 kg Körpergewicht eine Tagesdosis im Bereich zwischen 3 mg und 3000 mg, vorzugsweise zwischen 30 mg und 600 mg, Wirkstoff verabreicht.

Die nachfolgenden Beispiele illustrieren die oben beschriebene Erfindung; sie sollen jedoch diese in ihrem Umfang in keiner Weise einschränken. Temperaturen werden in Celsiusgraden angegeben. Alle Medien für Submers-Kulturen werden, wenn nicht anders angegeben, 20 Minuten bei 121° im Autoklaven sterilisiert, 10 Liter Fermentationsmedien 30 Minuten bei 134°.

Beispiel 1:

a) Herstellung einer Sporensuspension des Produzentenstammes. In einen 500 ml-Erlenmeyer-Kolben mit einer Schikane werden 100 ml des folgenden Hefe-Malz-Agar Nährmediums:

4 g Hefeextrakt; 10 g Malzextrakt; 4 g Glucose; 20 g Agar; pH 7; mit Wasser ad 1000 ml verdünnt

gegeben. Der Inhalt wird mit einer auf einem Schrägröhrchen gezogenen Kultur von Streptomyces antibioticus subs. antibioticus Tü 1718 angeimpft. Nach 2 Tagen Inkubation auf einer Schüttelmaschine (120 Umdrehung pro Minute, 5 cm Auslenkung, 27°C) wird 1 ml dieser Submerskultur auf 100 ml des schräg in einen Erlenmeyer-Kolben eingegossenen oben definierten Hefe-Malz-Agar-Mediums pipettiert. Nach 12 Tagen Inkubation bei 27° werden die Sporen mit 20 ml Tween-Wasser abgeschwemmt, durch Glaswolle filtriert und mit 20% Glycerin bei -22°C eingefroren. Die Sporendichte beträgt $1,5 \times 10^6$ Sporen/ml.

b) Herstellung einer Kultur bzw. Vorkultur des Produzentenstammes im Erlenmeyer-Kolben. Ein 500 ml Erlenmeyer-Kolben mit einer Schikane und 100 ml einer Nährlösung der Zusammensetzung, 2% Sojamehl, 2% Mannit, Wasser ad 100, pH 7,5, wird mit einer auf einem Schrägröhrchen gezogenen Kultur oder mit 0,2 ml Sporensuspension angeimpft und auf einer Schüttelmaschine mit 120 Umdrehungen pro Minute, 5 cm Auslenkung, bei 27°C inkubiert. Die maximale Antibiotikaproduktion ist nach ca. 80 Stunden erreicht. Zum Animpfen von 10, 20 und 25 Liter Fermentern genügt eine 45 Stunden alte Submerskultur aus der gleichen Nährlösung.

Beispiel 2: Ein Fermenter von 10 Liter Fassungsvermögen wird mit 9 Liter der im Beispiel 1 b) beschriebenen Nährlösung beschickt und 30 Minuten bei 134° sterilisiert. Sodann wird mit ca. 1000 ml einer gemäss Beispiel 1 b) hergestellten Submerskultur angeimpft, wobei folgende Fermentationsbedingungen eingehalten werden:

220 Umdrehungen pro Minute des Blattrührers, 5 Liter Luft pro Minute und 27°C. Soll der Fermenter Impfmaterial liefern, so wird nach 30 Stunden überimpft.

Beispiel 3: 10 Liter der gemäss Beispiel 2 hergestellten Kultur werden zum Impfen von 90 Litern der gemäss Beispiel 1 b) hergestellten Kultur in einem 100 Liter Fermenter verwendet. Sodann wird bis zur maximalen Antibiotikaproduktion (nach ca. 70 Stunden) bei 200 Umdrehungen pro Minute des Blattrührers, bei 40 Liter Luftzufuhr pro Minute und bei 27°C inkubiert.

Beispiel 4: Ein Fermenter von 20-25 Liter Fassungsvermögen enthaltend ca. 90% der in Beispiel 1b) verwendeten Nährlösung wird mit 10% des Nutzvolumens einer gemäss Beispiel 1b) hergestellten Vorkultur oder mit 40 ml einer gemäss Beispiel 1a) hergestellten Sporensuspension angeimpft. Die Fermentationsbedingungen sind:

800 Umdrehungen pro Minute, 10 Liter Luftzufuhr pro Minute, Temperatur 27°C. Nach ca. 30 Stunden hat die Kultur ihr pH-Minimum erreicht. Der im Verlauf der weiteren Fermentation wieder ansteigende pH-Wert wird dann durch eine pH-statische Regelung mit $H_2SO_4$ bei pH 6,5 festgehalten. Das Antibiotikum kann nach ca. 60-70 Stunden isoliert werden. Sofern der Fermenter zur Herstellung einer Vorkultur dient, wird nach 24 Stunden überimpft, wobei die Temperatur auf ca. 29°C erhöht wird.

Beispiel 5: Die Fermentation wird in einem 200 Liter Fermenter vorgenommen, wobei man 20 Liter der gemäss Beispiel 4 erhältlichen Vorkultur als Impfmaterial benutzt. Als Nährlösung benutzt man die in Beispiel 1b) verwendete. Die Fermentation wird bei einer Umdrehungszahl

von 800 Umdrehungen pro Minute, 100 Liter Luftzufuhr pro Minute und einer Temperatur von 28°C durchgeführt. Gegen Ende der Fermentation wird eine statische pH-Regelung vorgenommen, sodass der pH-Wert sich auf 6,5 einstellt. Die Antibiotika-Produktion ist nach ca. 50 Stunden beendet.

Beispiel 6: Herstellung von Mutanten des Stammes Tü 1718 von Streptomyces antibioticus subsp. antibioticus. Zu 9 ml Sporensuspension, wie in Beispiel 1 a) hergestellt, wird 1 ml eines Tris-Maleat Puffers pH 9, und 2 mg N-Methyl-N'-nitroso-N-nitro-guanidin gegeben. Die Inkubationstemperatur beträgt30°C und zunächst werden alle 3 Minuten, später alle 5 Minuten, 0,5 bzw. 1 ml Proben gezogen. Die Proben werden 2 Minuten in einer Eppendorf-Zentrifuge 3200 abzentrifugiert und zweimal mit physiologischer Kochsalzlösung gewaschen. Von jeder Probe werden Verdünnungsstufen gemacht und mit Top-Agar (0,8% Agar in physiologischer Kochsalzlösung) auf 40 ml Hefe-Malz-Agar-Platten gegossen. Nach 4 Tagen werden die Kolonien von Mutanten des Wildstammes vom 40 min.-Wert (0,1% Ueberlebensrate) auf neue Hefe-Malz-Agar-Platten überimpft. Nach 3 Tagen wird hiervon mit einem Korkbohrer (ø 5 mm) das Mycel ausgestochen und auf Testplatten gelegt. Die Auswertung erfolgt nach 24 Stunden.

Von den isolierten und ausgetesteten Einzelklonen werden diejenigen ausgewählt, die gleich viel oder mehr Antibiotikum (2S,5S)-2-(2-Hydroxyäthyl)-clavam produzieren wie der Wildstamm, was durch Testieren der Antibiotika-Wirkung, z.B. gegenüber Mucor miehei, oder auch dünnschichtchromatographisch möglich ist.

Beispiel 7: Eine gemäss einem der vorhergehenden Beispiele erhaltene Kulturbrühe wird zur Isolierung des Antibiotikums (2S,5S)-2-(2-Hydroxyäthyl)-clavams folgendermassen aufgearbeitet:

Zur Isolierung des gemäss einem der vorhergehenden Beispiele 2-6 in der Kultur produzierten Antibiotikums (2S,5S)-2-(2-Hydroxyäthyl)-clavams wird die Fermentationsbrühe durch eine Filterpresse unter Zusatz von 2% Celite filtriert. Das Mycel, das nur geringe antibiotische Aktivität aufweist, wird verworfen. Das Kulturfiltrat wird mit 5% granulierter Aktiv-Kohle 2 Stunden gerührt, danach abgepresst und anschliessend die Kohle ca. 1 Stunde mit 10% des Fermentationsgut-Volumens Aceton extrahiert. Der Aceton-Extrakt wird bis zum wässerigen Rückstand (ca. 1% des Fermentationsgut-Volumens) eingedampft und 5mal mit 0,5% des Fermentationsgut-Volumens Chloroform ausgeschüttelt. Nach dem Einengen der organischen Phase auf ein kleines Volumen erfolgt die Reinigung je nach Menge entweder über die präparative Dünnschichtchromatographie oder eine Kieselgelsäure der Korngrösse 0,04-0,063 mm (70-230 mesh). Als Fliessmittel wird Chloroform/Methanol 92:8 verwendet.

Aus dem so vorgereinigten Antibiotikum kann dieses folgendermassen in reinem Zustand erhalten werden:

a) Man chromatographiert an Kieselgel mit Chloroform/Methanol 9:1;
b) Man chromatographiert an Polyamid mit Chloroform;
c) Man führt eine Gel-Filtration an Sephadex LH 20 durch mit Chloroform als Lösungsmittel;
d) Man führt eine Craig-Verteilung im System n-Hexan, iso-Propyläther, Aceton, 0,1 M Phosphatpuffer pH 7 (8:25:25:25) aus.

Die Substanz ist nach einer Kombination der Reinigungsschritte a, b und c oder a und d rein.

Das so erhaltene (2S,5S)-2-(2-Hydroxyäthyl)-clavam fällt als farblose ölige Substanz an, die in Chloroform, Aceton, Methanol gut löslich und in Wasser mässig löslich ist. Die Substanz ist in lösungsmittelfreiem Zustand beschränkt stabil. Als Aufbewahrungsmittel eignet

sich Chloroform. In wässerigen Lösungen ist eine einigermassen gute Stabilität bei pH 5-7 gegeben. Bei der Dünnschichtchromatographie z.B. an Kieselgel-Alufolien kann die Substanz z.B. durch folgende Sprühreagenzien sichtbar gemacht werden: Ehrlich's Reagenz, rot; Vanillin/ $H_2SO_4$, lindgrün; Ninhydrin, gelb mit violettem Rand.

Die Rf-Werte in verschiedenen Laufmittelsystemen sind wie folgt:

| | |
|---|---|
| Chloroform-Methanol 7:3 | 0,65 |
| Chloroform-Methanol 92:8 | 0,43 |
| Dichlormethan-Propanol 92:8 | 0,40 |
| Propanol-Essigsäure-Wasser 3:1:1 | 0,65. |

Bei der Hochspannungselektrophorese kann erkannt werden, dass das Antibiotikum sowohl in saurem wie in alkalischem Milieu nicht protoniert bzw. deprotoniert wird. Das Molekül bleibt elektrisch neutral und läuft nicht.

$[\alpha]_D^{23}$ = - 202° ( c= 1% in Dimethylsulfoxid)

$[\alpha]_D^{23}$ = - 141° ( c= 0, 1% in Chloroform)

Zirkular - Dichroismus - Spektrum in Methanol:

$\lambda_{max}$ ($[\theta]^{25}$) = 203(+8300), 235 nm ( - 27 300).

Elementaranalyse des öligen bei 60° im Hochvakuum getrockneten Antibiotikums ergibt

C= 53,50%, H= 7,05%, N= 8, 91%, kein Schwefel nachweisbar.

Mol- Masse: 157 gemäss Feld - Ionisation - Massenspektrum.

Das Antibiotikum (2S,5S)-2-(2-Hydroxyäthyl)-clavam zeigt das in Fig.2 wiedergegebene, in Methanol aufgenommene UV-Spektrum, und das in Fig. 3 wiedergegebene in Abwesenheit von Lösungsmitteln aufgenommene Infrarotspektrum.

Beispiel 8: 1,0 g (2S,5S)-2-(2-Hydroxyäthyl)-clavam (6,36 mMol) werden in 80 ml abs. Methylenchlorid gelöst und mit 0,1 ml Bis-(tributylzinn)-oxid versetzt. Nach Zugabe von 1,2 ml (1,1 g) Phenylisocyanat wird die Reaktionslösung während 4 Stunden bei Raumtemperatur gerührt, wobei eine leichte Trübung auftritt. Nach erneuter Zugabe von Phenylisocyanat (0,2 ml; total 1,28 g; 10,76 mMol, 1,69 Aequivalente in Bezug auf das eingesetzte Substrat) wird während 15 Stunden auf 35°C erwärmt, worauf dünnschichtchromatographisch kein Ausgangsmaterial mehr nachgewiesen werden kann. Die Reaktionslösung wird nach dem Eindampfen zur Trockene in Aether aufgenommen und der ungelöste Rückstand von Diphenylharnstoff durch Filtration entfernt. Das eingedampfte Filtrat bestehend aus dem (2S,5S)-2-(2-Hydroxyäthyl)-clavam-phenylurethan wird mit dem analog hergestellten Rohprodukt ausgehend von 0,2 g (2S, 5S)-2-(2-Hydroxyäthyl)-clavam vereinigt und durch zweimalige Chromatographie gereinigt. Die Elution der mikrobiologisch aktiven Fraktionen erfolgt mit einem Lösungsmittelgemisch aus Methylenchlorid/Petroläther /2-Propanol im Verhältnis von 32:26:1. Nach dem ersten chromatographischen Reinigungsschritt resultiert eine angereicherte Fraktion des (2S,5S)-2-(2-Hydroxyäthyl)-clavam -phenylurethans, nach dem zweiten eine etwas kleinere Menge. Die Eluatfraktionen werden dünnschichtchromatographisch charakterisiert (auf MERCK Kieselgelplatten, Schichtdicke 0,25 mm). Die Entwicklung erfolgt entweder mit reinem Methylenchlorid oder Methylenchlorid/Petroläther/2-Propanol (16:13:1). Beim Besprühen mit Ehrlich Reagens werden einheitliche rote Substanzflecken erhalten. Mit Joddampf und im $UV_{254}$ sind auch nach dem zweiten chromatographischen Reinigungsschritt Flecken von Nebenkomponenten nachweisbar. Analytisch reines (2S,5S)-2-(2-Hydroxyäthyl)-clavam-phenylurethan wird durch präparative Hochdruck-Flüssigkeitschromatographie unter Verwendung einer ZORBAX ODS Säule (Fa. DUPONT, 21,2 mm I.D. x 25 cm) erhalten.

Mobile Phase: Methanol/Wasser (70:30)
Durchflussgeschwindigkeit: 17 ml/Min.
Druck 100 bar
Nachweis mit Ultraviolett-Detektor bei 210 nm.

Die angereicherte Substanz wird in einer Konzentration von 1% in reinem Acetonitril gelöst. Mit Hilfe einer Probeschlaufe von 1 ml Inhalt werden Mengen von 15 bis 30 mg pro Trennoperation auf die Säule gegeben. Das gewünschte Phenylurethan wird nach 5.38 Min. eluiert. Die Substanzpeaks aus den wiederholten Ansätzen werden in einem Fraktionensammler gesammelt und vereinigt. Das Methanol wird durch Konzentration im Rotationsverdampfer bei reduziertem Druck entfernt. Das wässrige Konzentrat wird lyophilisiert, wobei ein weisser flaumiger Rückstand erhalten wird.

Beispiel 9: Eine Lösung von 100 mg (0.64 mMol) (2S,5S)-2-(2-Hydroxyäthyl)-clavam in 20 ml Dichlormethan werden mit 66 mg (Dicyclohexylcarbodiimid) und 44 mg Phenoxyessigsäure versetzt und 5 Stunden bei 0°C gerührt. Es werden weitere 177 mg Dicyclohexylcarbodiimid und 132 mg Phenoxyessigsäure zugegeben und nach 16 Stunden 0.1 ml Pyridin. Nach weiteren 3 Stunden filtriert man den unlöslichen Rückstand ab und bringt das Filtrat im Vakuum zur Trockene. Der Rückstand wird an Kieselgel (PDC-Platte, 20 x 40 cm) mit Chloroform/Aceton (9:1) getrennt. Die im UV-Licht (254 nm) löschende Hauptzone enthält das (2S,5S)-2-(2-Phenoxyacetoxyäthyl)-clavam als farbloses Oel.
UV (MeOH): $\lambda_{max}$ ($\varepsilon$) = 276 (1500), 268 (1850), 264 sh, 217 (7600), 260 nm (8300).

IR (KBr): 1781, 1760, 1735 $cm^{-1}$ (CO-Valenzschwingungen).
$^1$H-NMR ($CDCl_3$): $\delta$ = 1.90 (mc, 8-$H_2$), 2.52/3.86 (3-$H_B$/3-$H_A$, AB-Teil von ABX-System, $J_{AB}$ = 11.5 Hz, $J_{AX}$ = 6.0, $J_{BX}$ = 7.0), 2.72/3.23 (6-$H_B$/6-$H_A$, AB-Teil von ABX-System, $J_{AB}$ = 17.0 Hz, $J_{AX}$ = 3,0, $J_{BX}$ 1.0), 4.24 (t, J = 7.0 Hz, 9-$H_2$), 4.55 (s, 11-$H_2$), 5.19 (d, J = 3.0 Hz, 5-H), 6.7-7.5 (komplex, 5-Aromat-H).

$R_F$ = 0.54 (DC-Kieselgel, Chloroform/10% Aceton; (2S, 5S)-2-(2-Hydroxyäthyl)-clavam: $R_F$ = 0.21
Anfärbung mit Ehrlichs Reagenz (rot).

Patentansprüche: (für alle benannten Länder ausser Oesterreich)

1. Das durch Züchtung des Stammes Streptomyces antibioticus subs. antibioticus Tü 1718 erhältliche Antibiotikum Tü 1718, seine Komponenten und Derivate.

2. Die Hauptkomponente $A_1$ des Antibiotikums gemäss Anspruch 1, das (2S,5S)-2-(2-Hydroxyäthyl)-clavam und seine Derivate der Formel

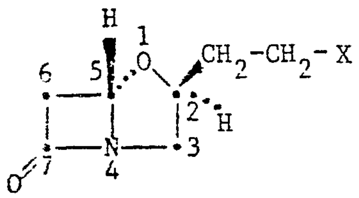

(IV)

in denen X eine freie oder funktionell abgewandelte Hydroxygruppe bedeutet, und gegebenenfalls Salze dieser Verbindungen.

3. Das (2S,5S)-2-(2-Hydroxyäthyl)-clavam der Formel

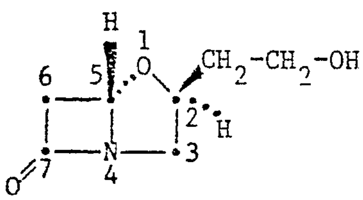

(I) .

4. Verbindungen gemäss Formel IV des Anspruchs 2, worin X eine mit einer Carbonsäure, Thiocarbonsäure, einer Sulfonsäure oder einer anorganischen Säure veresterte Hydroxylgruppe oder eine gegebenenfalls substituierte aliphatische, carbocyclische oder carbocyclisch-aliphatische Hydrocarbyloxy- oder Heterocyclyloxy-Gruppe mit 1-12 C-Atomen bedeutet.

5. Verbindungen gemäss Formel IV des Anspruchs 2, worin X eine Alkanoyloxygruppe mit 1-12 C-Atomen, die gegebenenfalls durch Halogenatome, Hydroxy-, Alkoxy- oder Aryloxygruppen substituiert sein kann,

oder eine 1-7 C-Niederalkoxycarbonyloxy- oder eine unsubstituierte oder im aromatischen Teil durch Methylgruppen, Aminogruppen oder Nitrogruppen substituierte Phenoxycarbonyloxy-Gruppe oder eine Cycloalkanoyloxygruppe mit 3-8 Ring-C-Atomen oder eine Cycloalkylniederalkanoyloxy-Gruppe mit 3-8 Ring C-Atomen und 1-4 C-Atomen im aliphatischen Teil, oder eine Heterocyclylcarbonyloxygruppe ist, wo der Heterocyclyl-Rest 2-, 3- oder 4-Pyridyl-, 2-Thienyl oder 2-Furyl ist.

6. Verbindungen nach Anspruch 2, worin X in Formel (IV) Acyloxy mit 1-12 C-Atomen oder monocyclisches aromatisches oder Niederalkylsulfonyloxy bedeutet, das unsubstituiert oder durch Halogen, Amino, Hydroxy oder Carboxy substituiert sein kann.

7. Verbindungen nach Anspruch 6, worin X in Formel (IV) Niederalkylcarbonyloxy bedeutet, worin "Niederalkyl" 1-7 C-Atome aufweist.

8. Verbindungen gemäss Formel IV des Anspruchs 2, worin X eine 1-7 C-Alkylurethan-Gruppe bedeutet.

9. Verbindungen gemäss Formel IV des Anspruchs 2, worin X eine unsubstituierte oder durch 1-2 Chlor- oder Bromatome substituierte Phenylurethangruppe bedeutet.

10. Eine Verbindung gemäss Anspruch 2, welche das (2S,5S)-2-(2-Phenoxyacetoxyäthyl)-clavam darstellt.

11. Pharmazeutisch verwendbare Salze einer Verbindung gemäss einem der Ansprüche 3-4, die im Kohlenwasserstoffrest des Aethers oder Esters eine salzbildende Gruppe enthält, und entsprechende Salze.

12. Verfahren zur Herstellung des Antibiotikums Tü 1718 des Anspruchs 1 und/oder der neuen Clavam-Verbindungen der Formel (IV) gemäss Anspruch 2, dadurch gekennzeichnet, dass man den Stamm Tü 1718 der Art Streptomyces antibioticus subsp. antibioticus oder eine sich von diesem Stamm ableitende, das Antibiotikum Tü 1718 produzierende Mutante, in einem eine Kohlenstoff- und Stickstoffquelle und anorganische Salze enthaltenden flüssigen Nährmedium aerob züchtet, bis die Nährlösung die gewünschte antibiotische Wirkung zeigt, wenn erwünscht, das entstandene Antibiotikum und/oder das darin enthaltene (2S,5S)-2-(2-Hydroxyäthyl)-clavam isoliert, und, wenn erwünscht, dieses in seine an der Hydroxygruppe funktionell abgewandelten Derivate, und diese gegebenenfalls in ihre Salze überführt.

13. Verfahren gemäss Anspruch 12, dadurch gekennzeichnet, dass man die Züchtung bei Temperaturen von 18-40° während 2-3 Tagen vornimmt, bis eine maximale Antibiotika-Produktion stattgefunden hat.

14. Verfahren gemäss einem der Ansprüche 12-13, dadurch gekennzeichnet, dass man bei der Produktions-Züchtung mit einer Vorkultur auf festem oder flüssigem Nährmedium oder mit einer Sporensuspension animpft.

15. Verfahren gemäss einem der Ansprüche 12-14, dadurch gekennzeichnet, dass man als assimilierbare Kohlenstoffquellen in der Nährlösung D-Glucose, D-Xylose, L-Arabinose, L-Rhamnose, D-Fructose, D-Galactose, D-Mannit oder Inosit verwendet.

16. Verfahren gemäss einem der Ansprüche 12-15, dadurch gekennzeichnet, dass man eine Nährlösung enthaltend ca. 2% Mannit und 2% Sojamehl verwendet.

17. Verfahren gemäss einem der Ansprüche 12-16, dadurch gekennzeichnet, dass man das Antibiotikum Tü 1718 aus dem Filtrat an Aktivkohle oder an einem makroretikulären Harz ohne funktionelle Gruppen (wie z.B. XAD-2, DIAION HP-20 und ähnliche) absorbiert, es daraus mit Aceton extrahiert, und aus dem im Acetonrückstand enthaltenen Antibiotikum

Tü 1718 die Hauptkomponente $A_1$ durch Chloroform extrahiert.

18. Verfahren gemäss Anspruch 17, dadurch gekennzeichnet, dass man die Hauptkomponente durch präparative Dünnschichtchromatographie oder Säulenchromatographie vorreinigt, und hierauf einer der folgenden Reinigungsoperationen unterwirft:
a) Chromatographie an Kieselgel mit Chloroform/Methanol 9:1
b) Chromatographie an Polyamid mit Chloroform
c) Gel-Filtration an Sephadex LH 20 mit Chloroform
d) Craig-Verteilung am System n-Hexan-iso-propyläther, Aceton, 0,1 M Phosphatpuffer pH 7 (8:25:25:25);
wobei die Schritte a, b und c oder a und d kombiniert werden.

19. Pharmazeutische Präparate enthaltend eine Verbindung gemäss einem der Ansprüche 5, 9 oder 10 zusammen mit einem pharmazeutischen Trägermaterial.

20. Pharmazeutische Präparate enthaltend die Verbindung des Anspruchs 3 oder eine Verbindung der Ansprüche 4, 6, 7, 8 oder 11, zusammen mit einem pharmazeutischen Trägermaterial.

21. Eine Verbindung gemäss einem der Ansprüche 3-4, 6,7,8 oder 11 zur Anwendung in einem Verfahren zur therapeutischen Behandlung des tierischen oder menschlichen Körpers, insbesondere als antifungisches Mittel.

22. Eine Verbindung gemäss einem der Ansprüche 5,9 oder 10 zur Anwendung in einem Verfahren zur therapeutischen Behandlung des tierischen oder menschlichen Körpers, insbesondere als antifungisches Mittel.

23. Der Stamm Tü 1718 von Streptomyces antibioticus subsp. antibioticus oder eine sich davon, das Antibiotikum Tü 1718 produzierende Mutante.

Patentansprüche: (für Oesterreich)

1. Verfahren zur Herstellung des Antibiotikums Tü 1718 und/oder der neuen Clavam-Verbindungen der Formel

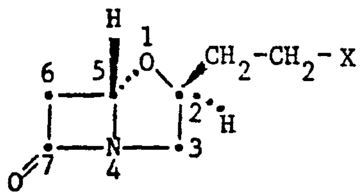

dadurch gekennzeichnet, dass man den Stamm Tü 1718 der Art Streptomyces antibioticus subsp. antibioticus oder eine sich von diesem Stamm ableitende, das Antibiotikum Tü 1718 produzierende Mutante, in einem eine Kohlenstoff- und Stickstoffquelle und anorganische Salze enthaltenden flüssigen Nährmedium aerob züchtet, bis die Nährlösung die gewünschte antibiotische Wirkung zeigt, wenn erwünscht, das entstandene Antibiotikum und/oder seine Komponenten, insbesondere seine Komponente $A_1$, das (2S,5S)-2-(2-Hydroxyäthyl)-clavam, isoliert, und, wenn erwünscht, dieses in seine an der Hydroxygruppe funktionell abgewandelten Derivate, und diese gegebenenfalls in ihre Salze überführt.

2. Verfahren nach Anspruch 1, dadurch gekennzeichnet, dass man das (2S,5S)-2-(2-Hydroxyäthyl)-clavam mit einer Carbonsäure, einer Thiocarbonsäure, einer Sulfonsäure oder einer anorganischen Säure verestert oder mit einem gegebenenfalls substituierten aliphatischen carbocyclischen oder carbocyclisch-aliphatischen oder heterocyclischen Alkohol mit 1-12 C-Atomen veräthert.

3. Verfahren nach Anspruch 2, worin die Estergruppe eine Alkanoyloxygruppe mit 1-12 C-Atomen, die gegebenenfalls durch Halogenatome, Hydroxy-, Alkoxy- oder Aryloxygruppen substituiert sein kann, oder eine 1-7 C-Niederalkoxycarbonyloxy- oder eine unsubstituierte oder im aromatischen Teil durch Methylgruppen, Aminogruppen oder Nitrogruppen substituierte Phenoxycarbonyloxy-Gruppe oder eine Cycloalkanoyloxygruppe mit 3-8 Ring-C-Atomen oder eine Cycloalkylniederalkanoyloxy-Gruppe mit 3-8 Ring-C-Atomen und 1-4 C-Atomen im aliphatischen Teil, oder eine Heterocyclylcarbonyloxygruppe ist, wo der Heterocyclyl-Rest 2-, 3- oder 4-Pyridyl-, 2-Thienyl oder 2-Furyl ist.

4. Verfahren nach Anspruch 3, worin die Estergruppe Acyloxy mit 1-12 C-Atomen oder monocyclisches aromatisches oder Niederalkylsulfonyloxy bedeutet, das unsubstituiert oder durch Halogen, Amino, Hydroxy oder Carboxy substituiert sein kann.

5. Verfahren nach Anspruch 4, worin die Estergruppe Niederalkylcarbonyloxy bedeutet, worin "Niederalkyl" 1-7 C-Atome aufweist.

6. Verfahren nach Anspruch 4, worin die Estergruppe eine 1-7 C-Alkylurethan-Gruppe bedeutet.

7. Verfahren nach Anspruch 4, worin die Estergruppe eine unsubstituierte oder durch 1-2 Chlor- oder Bromatomen substituierte Phenylurethangruppe bedeutet.

8. Verfahren nach Anspruch 4, worin die Estergruppe 2-Phenoxyacetoxy ist.

9. Verfahren nach Anspruch 1, dadurch gekennzeichnet, dass man das (2S,5S)-2-(2-Hydroxyäthyl)-clavam in solche Ester oder Aether überführt, die in der Ester bzw. Aetherkomponente eine salzbildende Gruppe aufweisen, und diese Ester oder Aether, wenn erwünscht, in entsprechende Salze überführt.

10. Verfahren gemäss einem der Ansprüche 1-9, dadurch gekennzeichnet, dass man die Züchtung bei Temperaturen von 18-40° während 2-3 Tagen vornimmt, bis eine maximale Antibiotika-Produktion stattgefunden hat.

11. Verfahren gemäss einem der Ansprüche 1-10, dadurch gekennzeichnet, dass man bei der Produktions-Züchtung mit einer Vorkultur auf festem oder flüssigem Nährmedium oder mit einer Sporensuspension animpft.

12. Verfahren gemäss einem der Ansprüche 1-11, dadurch gekennzeichnet, dass man als assimilierbare Kohlenstoffquellen in der Nährlösung D-Glucose, D-Xylose, L-Arabinose, L-Rhamnose, D-Fructose, D-Galactose, D-Mannit oder Inosit verwendet.

13. Verfahren gemäss einem der Ansprüche 1-12, dadurch gekennzeichnet, dass man eine Nährlösung enthaltend ca. 2% Mannit und 2% Sojamehl verwendet.

14. Verfahren gemäss einem der Ansprüche 1-13, dadurch gekennzeichnet, dass man das Antibiotikum Tü 1718 aus dem Filtrat an Aktivkohle oder an einem makroretikulären Harz ohne funktionelle Gruppen (wie z.B. XAD-2, DIAION HP-20 und ähnliche) absorbiert, es daraus mit Aceton extrahiert, und aus dem im Acetonrückstand enthaltenden Antibiotikum Tü 1718 die Hauptkomponente $A_1$ durch Chloroform extrahiert.

15. Verfahren gemäss Anspruch 14, dadurch gekennzeichnet, dass man die Hauptkomponente durch präparative Dünnschichtchromatographie oder Säulenchromatographie vorreinigt, und hierauf einer der folgenden Reinigungsoperationen unterwirft:

a) Chromatographie an Kieselgel mit Chloroform/Methanol 9:1
b) Chromatographie an Polyamid mit Chloroform
c) Gel-Filtration an Sephadex LH 20 mit Chloroform
d) Craig-Verteilung am System n-Hexan-iso-propyläther, Aceton, 0,1 M Phosphatpuffer pH 7 (8:25:25:25);

wobei die Schritte a, b und c oder a und d kombiniert werden.

16. Verfahren zur Herstellung von pharmazeutischen Präparaten dadurch gekennzeichnet, dass man eine Verbindung des Anspruchs 1 oder 2 oder eine Verbindung gemäss einem der Ansprüche 1, 3, 7 oder 8 mit einem pharmazeutischen Trägermaterial mischt.

17. Verfahren zur Herstellung von pharmazeutischen Präparaten dadurch gekennzeichnet, dass man eine Verbindung des Anspruchs 1 oder 2 oder eine Verbindung der Ansprüche 4, 5, 6 oder 9 mit einem pharmazeutischen Trägermaterial mischt.

18. Verfahren zur Herstellung einer Verbindung gemäss einem der Ansprüche 1, 2, 4, 5, 6 oder 9 zur Anwendung in einem Verfahren zur therapeutischen Behandlung des tierischen oder menschlichen Körpers, insbesondere als antifungisches Mittel.

19. Verfahren zur Herstellung einer Verbindung gemäss einem der Ansprüche 1, 3, 7 oder 8 zur Anwendung in einem Verfahren zur therapeutischen Behandlung des tierischen oder menschlichen Körpers, insbesondere als antifungisches Mittel.

20. Der Stamm Tü 1718 von Streptomyces antibioticus subsp. antibioticus oder eine sich davon, das Antibiotikum Tü 1718 produzierende Mutante.

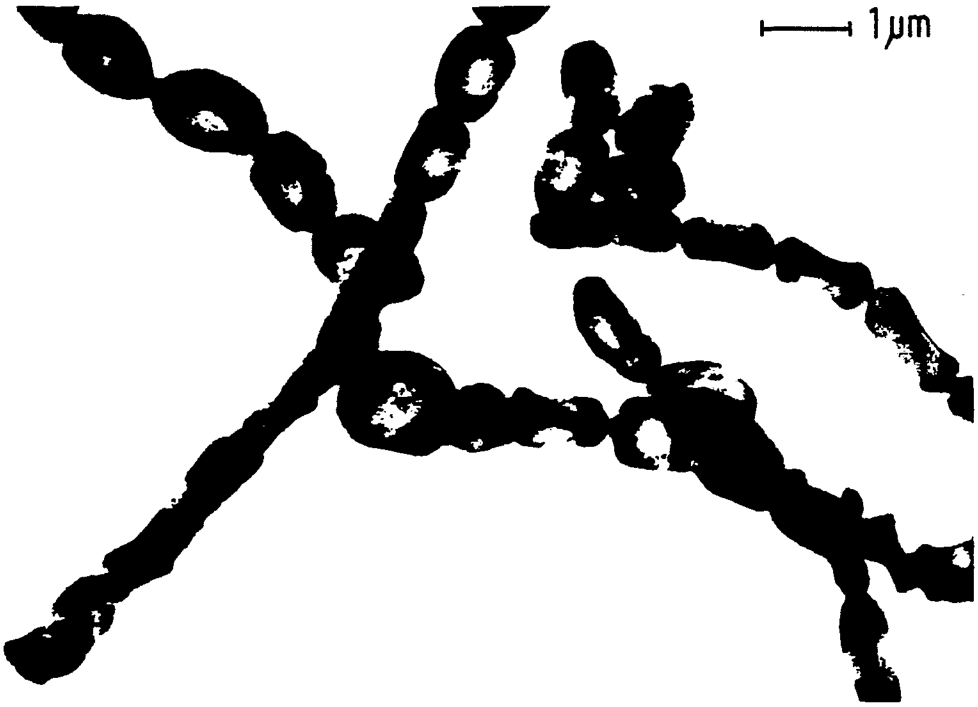


Fig. 1

Sporenketten von Streptomyces antibioticus Tü 1718

Fig. 2

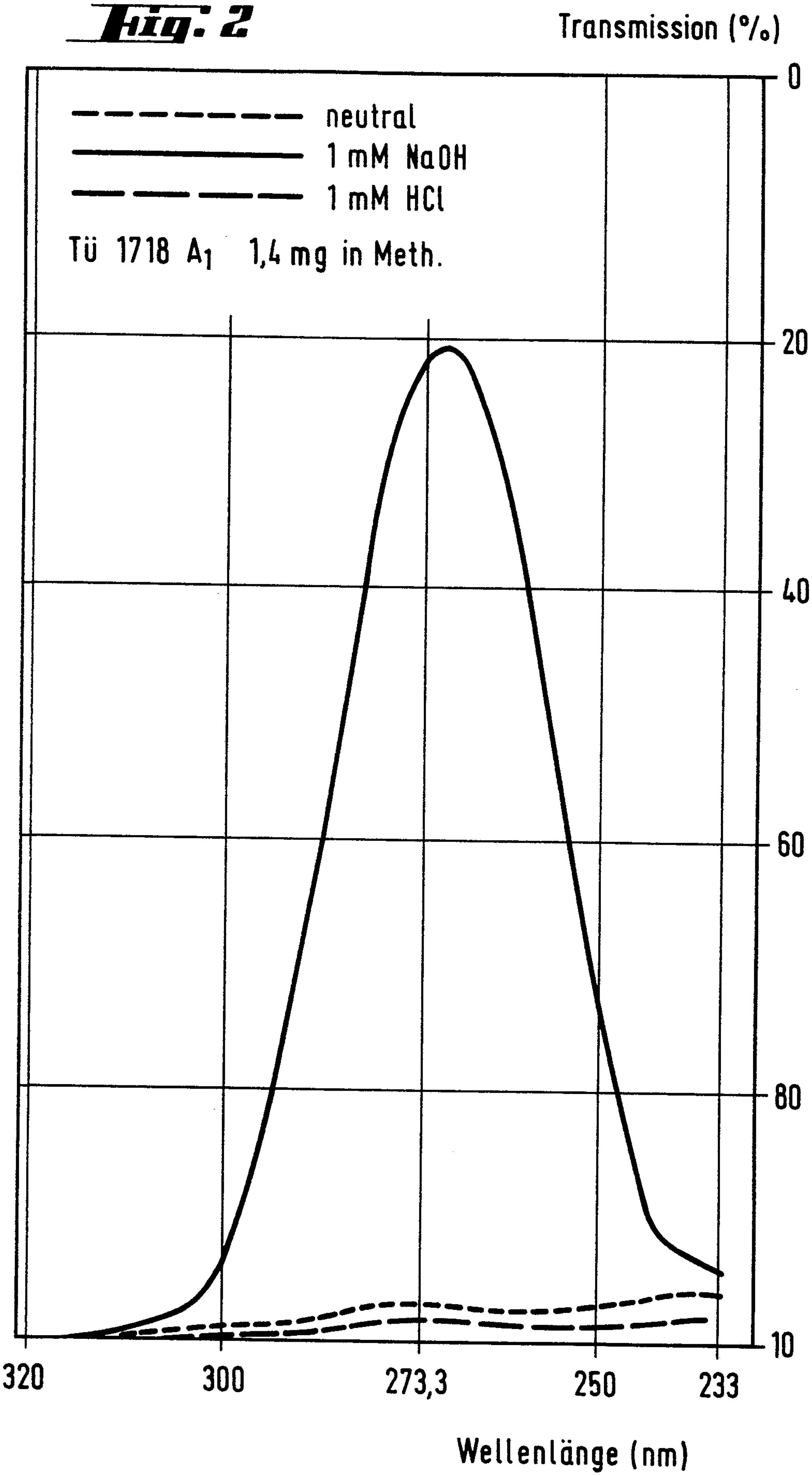
Transmission (%)
neutral
1 mM NaOH
1 mM HCl
Tü 1718 A1 1,4 mg in Meth.
0
20
40
60
80
10
320
300
273,3
250
233
Wellenlänge (nm)

Fig. 3

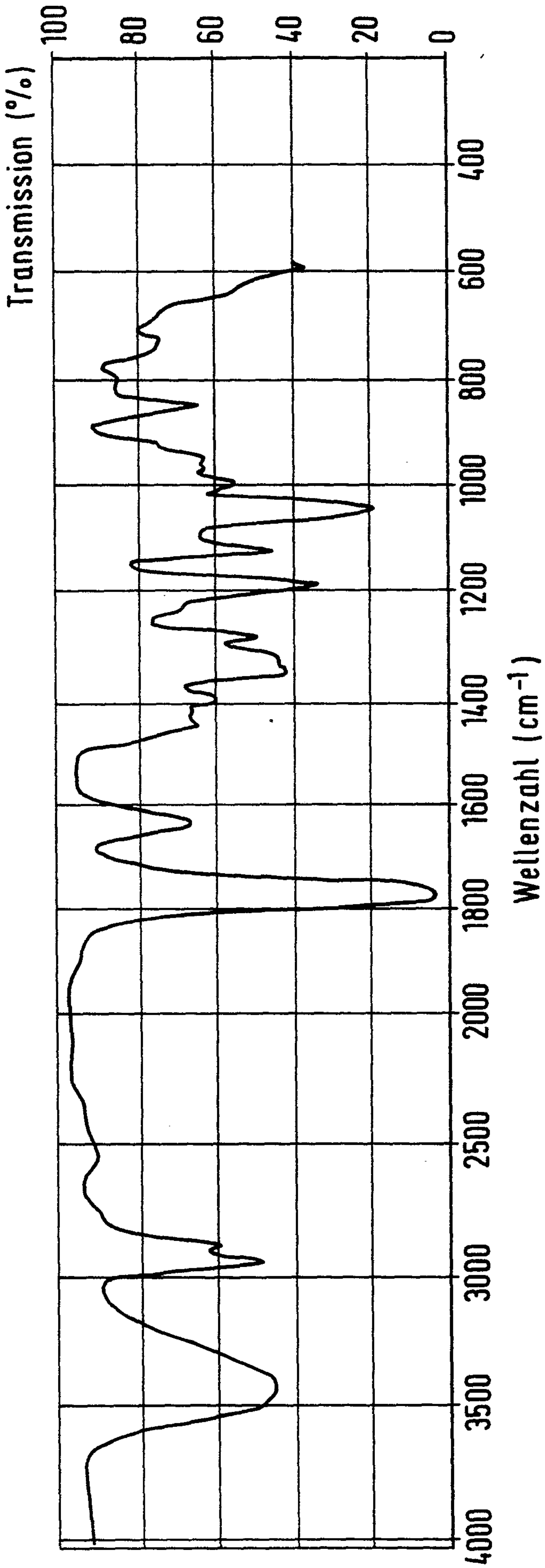
Transmission (%)
100
80
60
40
20
0
Wellenzahl (cm$^{-1}$)
400
600
800
1000
1200
1400
1600
1800
2000
2500
3000
3500
4000